# EUROPÄISCHE PATENTSCHRIFT

(11) **EP 0 957 944 B1**
(45) Veröffentlichungstag und Bekanntmachung des Hinweises auf die Patenterteilung: **06.05.2004**
(21) Anmeldenummer: 98961232.0
(22) Anmeldetag: 30.11.1998
(51) Int. Cl.: A61L 27/00, C12N 5/06

(54) **KNOCHENMATRIX, DEREN VERWENDUNG, THERAPEUTISCHE ZUBEREITUNG UND KIT**
BONE MATRIX, THE UTILIZATION THEREOF, THERAPEUTIC PREPARATION AND KIT
MATRICE OSSEUSE, SON UTILISATION, PREPARATION THERAPEUTIQUE ET NECESSAIRE ASSOCIE

(30) Priorität: 28.11.1997 DE 19752900
(43) Veröffentlichungstag der Anmeldung: 24.11.1999
(73) Patentinhaber: Gesellschaft für Biotechnologische Forschung mbH (GBF), 38124 Braunschweig (DE)
(72) Erfinder: MAYER, Hubert, D-38124 Braunschweig (DE); GROSS, Gerhard, D-38124 Braunschweig (DE); ROHDE, Manfred, D-38124 Braunschweig (DE); JÄGER, Volker, D-38124 Braunschweig (DE); SCHLIEPHAKE, Henning, D-30625 Hannover (DE); BARTHOLD, Marc, D-38124 Braunschweig (DE)
(74) Vertreter: Boeters, Hans Dietrich, Dr.
(86) Internationale Anmeldenummer: PCT/EP1998/007708
(87) Internationale Veröffentlichungsnummer: WO 1999/027977

(56) Entgegenhaltungen:
- EP-A- 0 358 506
- WO-A-95/32709
- WO-A-95/33821
- WO-A-98/00183
- WO-A-98/17791

## Beschreibung

Bisher werden vornehmlich Einzelkomponenten für Implantate eingesetzt. Einmal ist es synthetisches, anorganisches oder organisches Material. Des weiteren werden Wachstumsfaktoren eingesetzt. Es wird weiter xenotropes, jedoch kaum autologes biologisches Material als Implantatmaterial eingesetzt, was menschliches Gewebe oder Extrakte davon beinhaltet. Ex vivo gewonnene und in vitro expandierte Zellen werden weiter als autogenes Implantatmaterial benutzt.

Der frische autogene Knochen gilt heute nach wie vor als der Knochenersatz der ersten Wahl (Gross 1988, Mittelmeier et al. 1984, Schweiberer 1974). Die Entnahme körpereigenen Knochens ist allerdings mit einer nicht unerheblichen Erhöhung der perioperativen Morbidität der rekonstruktiven Eingriffe verbunden. So werden nach avaskulären Knochenentnahmen vom Beckenkamm permanente neurologische Ausfälle in bis zu 10% und persistierende Beschwerden in 10 - 28 % der Fälle beschrieben (Marx et al. 1988, Kurz et al. 1989, Fernyborough et al. 1992). Ähnliche Komplikationsraten sind für die Hebung revaskularisierter Transplantate berichtet worden (Duncan et al. 1985, Cohen et al. 1986, Jewer et al. 1991).

Zur Vermeidung einer autogenen Knochenentnahme werden heute verschiedene Wege beschritten, die sich auf die Induktion oder Regeneration von Knochengewebe am Ort des Bedarfes richten. Durch Verwendung von allogener Knochenmatrix konnte in Einzelfällen im Gesichtsbereich gute Rekonstitution erzielt werden (Kübeler et al. 1993). Eine breite Anwendbarkeit ist jedoch durch das hohe Infektionsrisiko insbesondere durch pathogene Viren nicht möglich. Weiter werden physikalische Impulse und biochemische Signale eingesetzt, um das Regenerationspotential des ortsständigen Knochengewebes zu stimulieren oder die Differenzierung nicht-osteogenen Regenerationsgewebes zu beeinflussen.

Die Simulation biochemischer Signale umfaßt vor allem die topische Applikation von Wachstums- und Differenzierungsfaktoren, um die Knochenneubildung zu initiieren. Durch die gentechnische Herstellung humanrekombinanter Proteinsequenzen stehen zahlreiche Faktoren heute als Reinsubstanzen zur Verfügung (Sampath et al. 1992, Takaoka et al. 1994, Wang et al. 1990). Allerdings haben sich viele dieser Moleküle wie der platelet derived growth factor (PDGF), die fibroblast growth factors (FGF) und die insulin like growth factors (IGF) im erwachsenen Organismus als vorwiegend allgemein proliferationsfördernd auf mesenchymale Gewebe erwiesen, ohne daß eine sichere Beeinflussung der Differenzierung nachweisbar gewesen wäre (Lynch et al. 1992, Becker et al. 1991, Schliephake et al. 1995). Nur eine Untergruppe der Superfamilie der transforming growth factors beta (TGFb), die bone morphogenetic proteins (BMPs), haben spezifische differenzierungslenkende Eigenschaften in Sinne einer osteoinduktiven Wirkung gezeigt (Reddi et al. 1993, Urist et al. 1984). Die Anwendung der Wachstumsfaktoren bei der Rekonstruktion des Gesichtsschädels beschränkt sich derzeit bis auf wenige Ausnahmen noch auf die experimentelle Chirurgie. Bei überwiegend positiven Berichten finden sich jedoch auch Arbeiten, die die Frage der Trägersubstanzen, der Dosierung und der Freisetzung dieser Moleküle in vivo problematisieren (Boyne 1996, Cook et al. 1994, Mayer 1996). Bei den derzeit verwendeten Mengen im Milligramm-Bereich wird bewußt eine starke Überdosierung der Faktoren angestrebt, um eine rasche Konzentrationsabnahme durch Auswaschung im Gewebe zu kompensieren (Boyne 1997). Untersuchungen mit human rekombinantem basic fibroblast growth factor an humanen Fibroblastenkulturen haben beispielsweise gezeigt, daß in vivo applizierte Mengen selbst im niedrigen µg-Bereich in vitro vermutlich durch toxische Wirkung die Zellproliferation komplett unterdrückt haben, während die in vitro optimal wirksamen Konzentrationen um eine Größenordnung von 10-3 niedriger als die in vivo eingesetzten lagen (Schliephake et al. 1997c). Ein dritter Weg zur Regeneration und Induktion von Knochengewebe wird durch die Möglichkeit eröffnet, knochenbildende Zellen in geringer Zahl mit minimal invasiven Methoden zu gewinnen, in vitro zu vermehren und zur Knochenbildung in den Spenderorganismus zu reimplantieren (Vacanti et al. 1993, Puelacher et al. 1997). Die Induktion von extrazellulärer Matrix in vitro ist bekannt (S. Kadiyla et al. 1997, Prockop 1997). Isolierung und Kultivierung der Stromazellen des Knochenmarks und die Wirksamkeit des Prinips wurden bei der Ratte etabliert, jedoch ist eine Übertragbarkeit auf den Menschen nicht so ohne weiters möglich. Die Schwierigkeit dieser als tissue engineering bezeichneten Vorgehensweise liegt darin, daß die Zellzahl an Osteoprogenitorzellen sehr gering ist und sie aus ihrem Gewebezusammenhang isoliert werden und somit den bisher noch weitgehend unbekannten gewebeeigenen Steuerungsmechanismen der Proliferation und endgültigen Differenzierung entzogen sind. Für die Fähigkeit der in vitro isolierten Zellen zur knöchernen Differenzierung spielt offenbar weiter das Alter des Individuums eine wichtige Rolle. Es wird dabei eine Reduktion der Fähigkeit zur Proliferation und osteogenen Differenzierung angenommen, die sich zumindest teilweise durch Supplementation mit Wachstumsfaktoren aufheben läßt. In menschlichen differenzierenden Osteoprogenitorzellen haben sich zwar eine Reihe von osteogenen Markermolekülen wie die alkalische Phosphatase, das Kollagen I und das Osteokalzin nachweisen lassen, die auf eine osteogene Differenzierung der Zellen hinweisen (Schliephake et al. 1996, Vilamitjana-Amedee et al. 1993, Faucheux et al. 1994). Da die isolierten und kultivierten Vorläuferzellen nicht als reine Kulturen anzusehen sind und sie möglicherweise einen pluripotenten Differenzierungsgrad aufweisen, ist damit zu rechnen, daß neben der Expression der morphologisch-biochemischen Charakteristika osteogener Zellen auch nicht osteogene Differenzierungspotentiale in der Zellkultur bestehen (Schliephake et al. 1997b, Prockop 1997), so daß nicht zuverlässig von der knochenbildenden Fähigkeit aller Zellen ausgegangen werden kann. Die erzielten Teilerfolge könnten weiter in nicht optimierten Zellkultivierungsbedingungen ihre Ursache haben. Bisher wurde einer optimalen Zellzüchtung nicht genügend Aufmerksamkeit geschenkt. Eine dreidimensionionale Fermentation von primären Knochenstammzellen im Reaktor ist bisher nicht beschrieben. Seit langen ist weiter grundsätzlich bekannt, daß das dreidimensionale Matrixgewebe zu einem höheren Grad an Differenzierung der integrierten Zellen beiträgt. Sie spielt dabei eine kombinatorische Rolle bei der Ausbildung der Differenzierung, einmal durch ihre deponierten Wachstumsfaktoren, zum anderen durch Komponenten, die mit der Zelloberfläche interagieren, beispielsweise Integrine (Damsky et al. 1992). Im Falle des Knochens kommt noch hinzu, daß noch weitgehend unbekannte nicht-kollagene Komponenten der Matrix, die als Nukleatoren und Antinukleatoren fungieren, eine notwendige Voraussetzung für eine geordnete und effektive Mineralisierung darstellen (Termine et al. 1980).

EP 0 358 506 beschreibt ein dreidimensionales Zell- und Gewebekultursystem, bei dem Zellen eines bestimmten Gewebes auf einer Stroma-Support-Matrix kultiviert werden, und zwar mit dem Ziel, die kultivierten Zellen bzw. die gesamte Matrix zu transplantieren. Die Stroma-Support-Matrix wird dadurch gewonnen, daß Stromazellen, wie beispielsweise Fibroblasten, auf einer dreidimensionalen Struktur präkultiviert werden, wobei diese Struktur aus verschiedenen Kunststoffen oder biologischen Materialien hergestellt wird, beispielsweise Katzendarmfäden oder Gelatine. Anschließend können die Zellen eines bestimmten Gewebes auf die Matrix inokkuliert werden. Als Quellengewebe werden Knochenmark, Haut, Leber, Pankreas, Niere und Nervengeweben erwähnt, nicht jedoch Knochengewebe selbst.

Stromazellen und die zu kultivierenden Gewebezellen können untereinander autogen oder allogen sein und ebenfalls autogen oder allogen zum Empfänger des Implantats.

Die Transplantation der kultivierten Zellen bzw. der gesamten Matrix erfolgt mit dem Zweck, das existierende Gewebe zu ersetzen oder zu verstärken. Das dreidimensionale Zell- und Gewebekultursystem kann weiter zum Screenen in vitro von beispielsweise zytotoxischen Substanzen oder Wachstums- und Regulationsfaktoren eingesetzt werden. EP 0 358 506 gibt damit keine Anregung, eine extrazelluläre Matrix (ECM) ohne künstliche Trägerstruktur zu synthetisieren.

In diesem Vorhaben soll deshalb über die bisher verfolgten Ansätze der direkten Einbringung von Zellen oder Wachstumsfaktoren oder ihrer Kombinationen ein neuer Weg beschritten werden und ein autogener Knochenersatz in vitro hergestellt werden.

So betrifft eine Ausführungsform der Erfindung eine Knochenmatrix, die dadurch gewinnbar ist, daß man
(a) von einem Explantat somatischer Zellen der Haut, des Knorpels, des Knochens oder des Zahnapparates ausgeht und osteogene Vorläuferzellen gewinnt
   oder von einer osteogenen Zellinie ausgeht,
(b) gegebenenfalls die gewonnenen osteogenen Vorläuferzellen oder die
   osteogene Zellinie in vitro expandiert,
(c) gemäß (a) gewonnene oder gemäß (b) expandierte osteogene Vorläuferzellen oder die gemäß (a) eingesetzte oder gemäß (b)
   expandierte Zellinie sich vermehren läßt und gegebenenfalls osteogene Zellen gewinnt,
(d) getrennt von Maßnahme (c) gemäß (a) gewonnene oder gemäß (b) expandierte osteogene Zellen oder die gemäß (a) eingesetzte oder gemäß (b) expandierte Zellinie in vitro extrazelluläre Matrix synthetisieren läßt und Knochenmatrix gewinnt,
(e) die synthetisierte Matrix gegebenenfalls von Zellen befreit und zellfreie Knochenmatrix gewinnt, und
(f) die gewonnene, gegebenenfalls zellfreie Knochenmatrix gegebenenfalls erneut mit osteogenen Zellen besiedelt, die gemäß (c) gewonnen worden sind, und durch osteogene Zellen besiedelte Knochenmatrix
   gewinnt.

Erfindungsgemäß kann Knochenmatrix dadurch gewinnbar sein, daß man bei Stufe (c) mit Hilfe
(i) einer Zugabe von Wachstumsfaktoren und/oder von Wachstumsfaktor-Rezeptoren und/oder
(ii) einer genetischen Transfektion mit Genen einer oder mehrerer der Komponenten gemäß (i) vermehrt.

Erfindungsgemäß kann Knochenmatrix dadurch gewinnbar sein, daß man als Wachstumsfaktoren gemäß (i) Fibroblast Growth Faktors (FGFs), Epidermal Growth Faktors (EGFs), insbesondere VEGFs, oder Parathhormone (PTHs) und/oder als Wachstums-Rezeptoren FGFRs, VEGFRs oder PTHRs und/oder gemäß (ii) deren Gene verwendet.

Erfindungsgemäß kann Knochenmatrix dadurch gewinnbar sein, daß man bei Stufe (d) mit Hilfe
(i) einer Zugabe von Wachstumsfaktoren, insbesondere Bone Morphogenetic Proteins (BMPs), und/oder Wachstumsfaktoren-Rezeptoren, insbesondere BMPRs, und/oder BMPR-Signalmolekülen (Smads) und/oder
(ii) einer genetischen Transfektion mit Genen einer oder mehrerer der Komponenten gemäß (i) extrazelluläre Matrix synthetisiert.

Erfindungsgemäß kann Knochenmatrix dadurch gewinnbar sein, daß man bei Stufe (e) die die Knochenmatrix besiedelnden Zellen beläßt oder in ihrer Vermehrung behindert, insbesondere durch Bestrahlung oder durch chemische Behandlung.

Erfindungsgemäß kann Knochenmatrix dadurch gewinnbar sein, daß man bei Stufe (e) die die Knochenmatrix besiedelnden Zellen entfernt, indem man die Zellen enzymatisch ablöst oder einfriert und durch Waschen entfernt.

Erfindungsgemäß kann Knochenmatrix dadurch gewinnbar sein, daß man bei Stufe (f)
(i) zellfreie Knochenmatrix, die mit Hilfe von autogenen osteogenen Vorläuferzellen des Menschen gewonnen worden ist, mit osteogenen Zellen xenogener oder allogener Herkunft besiedelt oder
(ii) zellfreie Knochenmatrix, die mit Hilfe von xenogenen oder allogenen osteogenen Vorläuferzellen gewonnen worden ist, mit osteogenen Human-Zellen autogener Herkunft besiedelt oder
(iii) zellfreie Knochenmatrix, die mit Hilfe von autogenen osteogehen Vorläuferzellen des Menschen gewonnen worden ist, mit osteogenen Human-Zellen autogener Herkunft besiedelt.

Erfindungsgemäß können Knochenmatrix und/oder osteogene Zellen dadurch gewinnbar sein, daß man bei Stufe (a) von Explantaten des Beckenkamms oder von osteogenen Zellinien ausgeht.

Eine weitere Ausführungsform der Erfindung betrifft eine therapeutische Zubereitung, gekennzeichnet durch eine Knochenmatrix gemäß der Erfindung.

Die erfindungsgemäße Zubereitung kann in Form einer Salbe, einer Emulsion oder eines Pflasters vorliegen.

Eine weitere Ausführungsform der Erfindung betrifft einen Kit, gekennzeichnet durch eine Knochenmatrix mit osteogenen Zellen gemäß der Erfindung zum Testen von exogenen Faktoren, die die Bildung und Differenzierung von Zellen beeinflussen.

Schließlich betrifft eine Ausführungsform der Erfindung eine Verwendung von Knochenmatrix und/oder osteogenen Zellen gemäß der Erfindung zur Herstellung eines autogenen, xenogenen oder allogenen Implantatmaterials.

Erfindungsgemäß kann also in einem ersten Schritt eine autogene dreidimensionale Matrix in vitro produziert und in einem zweiten Schritt neubesiedelt werden. Durch genetische Veränderung der Produktionszellen kann ihre Wirkungsweise gezielt verbessert werden.

Ein neues hier beschriebenes Verfahren ist Gegenstand der Erfindung und beinhaltet die getrennte Herstellung einzelner Komponenten, einmal einer biologisch aktiven Matrix und zum anderen der lebenden Zellen und deren Kombinationen und deren Nutzung, d.h. der Gesamtheit und ihrer Einzelkomponenten insbesonders zur Verwendung als autologer zellbeladener gewebetechnologisch hergestellter Knochenersatz.

Dabei kann folgender neuer Ansatz verfolgt werden: Es werden nicht Einzelkomponenten, wie Wachstumsfaktoren, Matrix oder Zellen einzeln benutzt, sondern ein gewebetechnologisch hergestelltes zellhaltiges autogenes Material hergestellt. Um dies zu erreichen, werden einzelne Komponenten getrennt optimiert, einmal die biologisch aktive Matrix und zum anderen die lebenden Zellen, und anschließend vereinigt zur Herstellung einer dreidimensionalen Knochenmatrix mit integrierten lebenden Zellen. Dabei werden insbesonders menschliche Stammzellen aus dem Beckenkamm isoliert und in Zellkultur vermehrt. Durch Teilen der Zellen in zwei Pools werden Zellzahl einerseits und Matrixsynthese andererseits getrennt optimiert durch exogene Zugabe von distinkten Faktoren und/oder durch Expression von autokrinen Differenzierungsfaktoren bzw. deren Rezeptoren mittels gentechnischer Methoden. Zur Synthese der Matrix können auch etablierte menschliche Zellinien verwendet werden. Der Vorteil dieses neuartigen Verfahrens liegt nun darin, daß die Zellen, die Matrix synthetisierten und am Ende ihrer Vitalität sind, entfernt werden können und die Matrix durch vitale Zellen neubesiedelt werden kann, und zwar entweder durch autologe oder genetisch veränderte Zellen. Nach dieser Neubesiedlung kann der Zellzustand, wie proliferierend, differenzierend, mineralisierend, durch Kulturführung eingestellt werden und nach Implantation der optimale Zustand der Zellen und der Matrix für die Knochenneubildung in vivo ermittelt werden. Die Wirkung dieses zellbeladenen gewebetechnologisch hergestellten Knochenersatzes auf Knochenneubildung wird in athymischen Ratten durch Implantation in subkutanes Muskelgewebe sowie in Kontinuitätsdefekten des Femurs überprüft. Um nach Implantation die Donorzellen und ihre Wirkung verfolgen und sie von den Wirtszellen und ihrer Wirkung unterscheiden zu können, werden sie mit einem TAG-Gen (Green light fluorescence protein bzw β-Galaktosidase) tranfiziert und damit sichtbar gemacht. Durch vergleichende Untersuchungen in vitro einerseits und in vivo andererseits soll eine mögliche Abhängigkeit der Knochenbildung der Donorzellen überprüft werden hinsichtlich des Spenders, der Zellzahl, des Differenzierungszustandes, der Expression von autokrinen Wachstumsfaktoren bzw. deren Rezeptoren und der dreidimensionalen autologen Matrix. Die Analyse soll immunhistochemisch und mittels RT-PCR von relevanten Genen und durch Fluoreszenz und atomaren Nachweis im Fall des Calciums erfolgen. Nach erfolgreicher Testung im xenogenen Implantationsmodell wird ein autogenes Reimplantationsmodell mit osteogenen Zellen des Beckenkammes bei adulten Schafen erprobt. Dabei wird gewebetechnologisch hergestellter Knochen in nicht heilende Kontinuitätsdefekte des Unterkiefers eingesetzt. Das beschriebene Verfahren wird als Vorraussetzung für Knochenersatz allgemein und insbesondere im Kieferbereich beim Menschen mit gewebetechnologisch hergestellem Knochenersatzmaterial angesehen.

Erfindungsgemäß können folgende Maßnahmen vorgesehen werden bzw. die getrennte Herstellung folgender einzelner Komponenten:
1. die kontrollierte Synthese der Matrix in vitro,
2. das kontrollierte Wachstum von Zellen in vitro,
3. die anschließende Kombination der Matrix und der Zellen in vitro,
4. autogene und allogene Nutzung der Einzelkomponenten und deren Kombinationen in der Herstellung der Gesamtkomponenten in einem breiten Ausmaß.

Die Herstellung kann folgende Schritte beinhalten:
1. Isolierung von Zellen, wobei menschliche somatische Zellen, nämlich Zellen der Haut, des Knorpels, des Knochens oder des Zahnapparates, aus Explantaten im Vordergrund stehen und deren direkte Nutzung. Es können auch Zellinien verwendet werden.
2. Die Zellen können gesund, krank und genetisch modifiziert sein.
3. Expansion dieser Zellen in vitro im Kleinmaßstab bzw. im Fermenter und Induktion von Differenzierungsvorgängen der Zellen und deren Nutzung.
4. Herstellen von biologisch aktiver zwei- bzw.dreidimensionaler Matrix durch diese expandieren Zellen in vitro und deren direkte Nutzung.
5. Entfernen der Zellen und direkte Nutzung der zellfreien Matrix.
6. Neubesiedlung der Matrix durch Zellen und deren Nutzung.
7. Induktion von Differenzierungsvorgängen und Wechselwirkungsbeziehungen zwischen der biologisch aktiven Matrix und den lebenden Zellen und deren Nutzung.

Die Nutzung der hergestellten biologisch aktiven Matrix kann im Einzelnen folgendes beinhalten:
1. Äußere Applikation wie bei Haut- oder Zahnerkrankungen und für kosmetische Zwecke direkt und in Kombination mit anderem in Form beispielsweise von Salben, Emulsionen, Pflastern.
2. Nutzung als autogenes Implantatmaterial.
3. Nutzung als allogenes Implantatsmaterial.
3. Nutzung in Kombination mit anderen bekannten Materialien als Implantatmaterial, insbesondere bei Knochen-, Knorpel- bzw. Zahnimplantaten.
4. Nutzung als Trägermaterial zur Neubesiedlung durch Zellen, insbesondere autogene und allogene Zellen.

Die Nutzung der hergestellten biologisch aktiven Zellen kann im Einzelnen folgendes beinhalten:
1. Nutzung als autogenes Implantatmaterial insbesondere im Haut-, Knochen-, Knorpel- bzw. Zahnbereich.
2. Nutzung als allogenes Implantatsmaterial.
3. Nutzung als Testsystem in folgender Kombination.
   - Matrix von gesunden Zellen mit neubesiedelten gesunden Zellen
   - Matrix von gesunden Zellen mit neubesiedelten kranken Zellen
   - Matrix von kranken Zellen mit neubesiedelten kranken Zellen
   - Matrix von kranken Zellen mit neubesiedelten gesunden Zellen
   - Matrix von genetisch modifizierten gesunden und kranken Zellen
   mit gesunden, kranken und genetisch modifizerten gesunden und
   kranken Zellen, unter Benutzung der Testparameter, wie beispielsweise Zellmorphologie, Zelladhäsion, Zellwanderung, Zelldifferenzierung, Matrixsynthese mit Hilfe beispielsweise enzymatischer, histologischer und genetischer Methoden.
4. Nutzung als Testsystem der Einzelkomponenten wie der Kombinationen zum Testen des Einflusses von exogen zugegebenen Testsubstanzen.

### Erläuterndes Beispiel 1:

Osteogene Vorläuferzellen insbesondere des Menschen konnten aus Explantaten in geringer Anzahl gewonnen und in vitro expandiert werden. Nach Teilung des ursprünglichen Zellpools konnten die Komponente der Matrix und die Komponente der Zellen wie nachfolgend erläutert getrennt hergestellt werden. Durch exogene Zugabe einerseits und/oder genetische Transfektion andererseits von entsprechenden Wachstumsfaktoren (FGF, VEGF, PTH) bzw. ihrer Rezeptoren (FGFR, VEGFR, PTHR) konnten osteogene Vorläuferzellen dazu veranlaßt werden, sich vor allem zu vermehren. Durch exogene Zugabe einerseits und genetische Transfektion andererseits von entsprechenden Wachstumsfaktoren (BMPs) bzw. ihrer Rezeptoren (BMPRs und/oder deren Signalmolekülen, Smads) konnten osteogene Vorläuferzellen dazu veranlaßt werden, unter bekannten Bedingungen vor allem extrazelluläre Matrix zu synthetisieren. Nach der Matrixsynthese konnten die Zellen auf unterschiedliche Weise entweder durch enzymatischen Verdau, durch mechanisches Abkratzen oder durch Einfrieren, sowie DNA und RNA durch enzymatischen Verdau degradiert und durch Waschen entfernt werden. Die Matrix, die Kollagene und nichtkollagene Komponenten enthält, die einen komplexen Einfluß auf Zellen ausüben, konnte in unterschiedlicher Weise nachgewiesen werden. Danach wurde die Matrix mit Zellen neu besiedelt. Ein erneutes Ausplattieren beispielsweise von Zellen des restlichen Ausgangspools auf die Matrix führte zu einer stark erhöhten Adsorption der Zellen, zu einem versärkten Wachstum und zu einer zeitlichen Verkürzung der Zelldifferenzierung im Vergleich zur Kontrolle ohne Matrix. Die Differenzierungs-Mineralisierungphase verkürzte sich von 12 bis 15 Tagen auf 4 bis 6 Tage. Weiterhin zeigte sich diese Wirkung auf xenogene osteogene Vorläuferzellen. So hat beispielsweise die Matrix von osteogenen Zellen der Maus eine Wirkung auf osteogene Zellen des Menschen und des Hühnchens vergleichbar zur eigenen Matrix. Weiterhin zeigte sich, daß die extrazelluläre Matrix von beispielsweise BMP2 bzw. BMPR Typ I exprimierenden Zellen eine verstärkte Wirkung auf osteogene Zellen beispielsweise der Menschen und des Hühnchens hat. Dies zeigt, daß Matrixsynthese und Zellvermehrung getrennt vorgenommen werden können und damit autogene und xenogene Kombinationen von Matrix mit Zellen möglich sind, woraus sich entsprechend neuartige und erweiterte Anwendungen ergeben.

### Erläuterndes Beispiel 2:

Einfluß von nativer extrazellulärer Matrix ( ECM) auf Differenzierung und Mineralisierung von mesenchymalen Stammzellen unter osteoinduktiven Bedingungen in vitro

Der Ansatz für die durchgeführten Versuche war die Produktion von extrazellulärer Matrix (ECM) mittels Zellinien, die osteogene, fibroblastoide und Eigenschaften haben. Als Produktionszellen wurden verschiedene humane (SAOS 2, h FOB) und murine Zellinien ( C3 10T1/2), (C 310T1/2 Derivate: BMP2,5,7, BMP Rezeptor ALK I und ALKI dominant negativ ), LTK- getestet. Als Vergleichszelllinie wurde eine Nierenzelllinie verwendet (BHK).
Die Zellen wurden nach Erreichen der Konfluenz fünf Tage unter Zugabe von osteoinduktiv wirkenden -Glycerophophat und Ascorbat kultiviert. Danach wurde mit Ethanol fixiert bzw. die verschiedenen Lysisverfahren angewendet. Zur Zellyse wurden die bekannten Detergenzien Tween-20, Triton-X-100 in einer Konzentration von 0,25 % für 1 h sowie das denaturierende Agens Guanidinium-Hydrochlorid in einer Konzentration von 4M für ½ h verwendet.

Ein weiteres Verfahren beruht auf einer Kombination aus zwei Puffern mit verschiedenen Salzkonzentrationen (hypertonischer und hypotonischer Puffer). Durch aufeinaderfolgende Inkubation werden die Zellen durch den eigenen osmotischen Druck lysiert. Die Wirkung der von den Zellinien produzierten extrazellulären Matrix wurde anhand von mesenchymalen Stammzellen (MSCs) getestet, die aus der Tibia von Hühnerembryonen isoliert wurden (Testzellen). Dazu wurden die MSCs auf den ethanolfixierten bzw lysispuf-ferbehandelten Zellen unter denselben osteoinduktiven Bedingungen kultiviert und der Anstieg der Alkalischen Phosphatase und der Mineralisierungs als Differenzierungsparameter untersucht.

Die Aktivität der ALP wurde quantitativ und histochemisch nach Standartverfahren überprüft. Der Mineralisierungsgrad der extrazellulären Matrix wurde mit Hilfe von Calcein gemessen, das mit dem extrazellulären Calcium einen fluoreszierenden Komplex bildet. Mit Hilfe eines Fluorimeters kann die Fluoreszenz gemessen und eine relative Aussage über die Mineralisierung gemacht werden.

Als Kontrolle wurden die Produktionszellen und die MSCs separat auf ihre Mineralisierung auf unbehandeltem und gelantinebeschichtetem Untergrund untersucht.
Besonders gute Ergebnisse konnten mit der menschlichen Tumorzellinie Saos-2 erzielt werden. Auf den ethanolfixierten Zellen dieser Linie erreichten die MSCs nach Tagen 5 eine signifikante Steigerung der ALP und eine mehr als zehnfache Steigerung der Mineralisierung. Während auf den lysisbehandelten Produktionszellen zwar ein Rückgang der Mineralisierung der MSCs gegenüber denen auf ethanolfixierten Saos-2 zu beobachten war, konnte allerdings ein klarer Anstieg in der ALP - aktivität und Mineralisierung gegenüber den MSCs auf gelantinebeschichtetem und unbehandeltem Kultivierungsmaterial verzeichnet werden.
Im Vergleichsversuch zeigten MSCs ohne Matrixmaterial erst nach zehn Tagen ein Maximum der Mineralisierung, die etwa fünffach gegenüber dem Tage der Aussaat erhöht ist.
Auch mit den anderen untersuchten Zellinien (C3H10T1/2 und Derivate, LTK-), bei denen es sich um murine fibroblastoide Zellinien handelt, konnte eine signifikante Steigerungen der ALP- aktivität und Mineralisierung der MSCs sowohl auf ethanolfixierten als auch auf lysispufferbehandelten Zellen beobachtet werden. Mit der BHK Zelllinie konnte keinerlei stimulierender Effekt gemessen werden. Die Produktionszellen alleine zeigten unter diesen Versuchsbedingungen keine signifikannte ALP-aktivität und Mineralisierung.

Ein Vergleich der angewendeten Lysisverfahren zeigte, daß die Aktivitätsverluste der von den Zellinien produzierten Matrix umso geringer waren desto schonender die verwendete Lysismethode war. Von den getesteten Methoden erwies sich die Kombination aus hypertonischem und hypotonischem Puffer als der schonendste Weg die Zellen aufzuschliessen, gefolgt von der Behandlung mit Tween-20, während sich die Behandlung mit Triton-X-100 und dem denaturierenden Guanidinium-Hydochlorid als sehr drastische Lysismethoden erwiesen haben.
Die durchgeführten Experimente zeigen, daß es möglich ist, native extrazelluläre Matrix durch Produktionszellen zu produzieren, die Zellen gegebenenfalls abzutöten oder sie zu entfernen und stimulierende Effekte der ECM auf neu besiedelte Zellen wie mesenchymalen Stammzellen (MSCs) hinsichtlich der Differenzierung und Mineralisierung auszuüben.

### LITERATUR

1. BARRREILLE, R., LAFAGE, M.H., WENT, R., DARD, M., BAQUEY, CH.,
   AMEDEE, J.
   Specific analysis of newly formed bone into hydroxyapatite endobon® material loaded with human bone marrow stroma cells implanted in extraosseous sites.
   Abstract Proceedings of ESB 1997.
2. BECKER, W., LYNCH, S.E, LEKHOLM, U., BECKER, B.E., CAFFESSE, R., DONATH, K., SANCHEZ, R.
   A comparison of ePTFE membranes alone or in combination with platelet derived growth factors and insulin-like growth factor - I or demineralized freeze-dried bone in promoting bone formation around immediate extraction socket implants.
   J Periodontol, 1992, 63: 929 - 940
3. BOYNE, P. J., G. O. KRÜGER
   Fluorescence microscopy of alveolar bone repair.
   Oral Surg Oral Med Oral Path, 1962, 15: 265 - 281
4. BOYNE, P.J.
   Animal studies of application of rhBMP-2 in maxillofacial reconstruction.
   Bone, 1996, 19: 83S-92S.
5. BUSER, D., HIRT, H.-P., DULA, K., BERTHOLD, H.
   Gleichzeitige Anwendung von Membranen bei Implantaten mit periimplantären Knochendefekten.
   Schweiz Monatsschr Zahnmed 102, 1491 (1992).
6. COHEN, S.R., SHAW, W.W., MCCARTHY, J.
   Review of the morbidity of 300 free-flap donor sites.
   Plast Reconstr Surg 1986, 77: 948 - 953.
7. DAHLIN, C., LINDE, A., GOTTLOW, J., NYMAN, S.
   Healing of bone defects by guided tissue regeneration.
   Plast Reconstr Surg 81: 672, 1988.
8. DINER, P.-A., KOLLAR, E.-M., MARTINEZ, H., VAZQUEZ, M.-P.
   Intraoral distravction for mandibular lenghtening. a technical innovation
   J Craniomaxillofac Surg 1996, 24: 92 - 95.
9. DUNCAN, M.J., MANKTELOW, R.T., ZUKER, R.M., ROSEN, J.B.
   Mandibular reconstruction ib the radiated patient. The role of osteocutaneous free tissue transfers.
   Plast Reconstr Surg 1985, 76: 829 - 840.
10. FAUCHEUX, C., BAREILLE, R., ROUAIS, F., AMEDEE, J., LIEBENDÖRFER, A., DARAD, M.
   Biocompatibility testing of a bovine hydroxy-apatite ceramic materiual with the use of osteo-progenitor cells isolated from human bone marrow'
   J Mater Sci Mater Med, 1994, 5: 635 - 639
11. FERNYHOUGH, J.C., SCHIMANDLE, J.J., WEIGEL, M.C., EDWARDS, C.C., LEVINE, A.M.
   Chronic donor site pain complicating bone graft harvesting from the posterior iliac crest for spinal fusion
   Spine 1992, 17: 1474-1480
12. GROSS, U. M.
   Biocompatibility - The interaction of biomaterials and host response. J Dent Educ, 1988, 52: 798 - 803
13. JEWER, DD:, NOYD, B., MANKTELOW, R.T., ZUKER, R.M., ROSEN I.B., GULLANE, P.J., ROTSTEIN, L.E., FREEMANN, J.E
   Orofacial and mandibular reconstruction with the iliac crest free flap: A review of 60 cases and a new method of classification.
   Plast Reconstr Surg 1989, 84:391 - 404.
14. KLEIN, C.
   Die Knochenverlängerung nach Ilizarov zur Behandlung der mandibulären Mikrognathie im Kindesalter
   Fortschr Kiefer Gesichtschirurgie 1994, 39: 150 - 152.
15. KRAMER, F.J.
   Healing of critical size defects of rat calvaria
   Inauguraldissertation, Medizinische Hochschule Hannover 1997
16. KURZ, L.T., GRAFIN, S.R., BOOTH, R.E.
   Harvesting autogenous iliac bone grafts
   Spine 1989, 14: 1324-1331
17. LYNCH, S.E., BUSER, D., HERNANDEZ, R.A., WEBER, H.P., SUICH, H., FOX, C.H., WILLIAMS, R.C
   Effects of the platelet-derived growth factor / insulin-like growth factor-I combination on bone regeneration around titanium dental implants. Results of a pilot study in beagle dogs
   J Periodontol, 1991, 62: 710 -716
18. MARX, R.E., MORALES, M.J.
   Morbidity from bone harvest in major jaw reconstruction
   J Oral Maxillofacial Surg 1988, 46:196-203
19. MAYER, M., HOLLINGER, J., RON, E., WOZNEY, J.
   Maxillary alveolar cleft repair in dogs using recombinant human bone morphogenetic protein-2 and a polymer carrier.
   Plast Reconstr Surg 1996, 98: 247
20. MCCARTHY, J.G., STAFFENBERG, D.A., WOOD, R.J., CUTTING, C.B., GRAYSON, B.H., THORNE, C.H.
   Introduction of an intraoral bone-lenghtening device
   Plast Reconstr Surg 1995, 96: 978 - 981.
21. MITTELMEIER, H., B. D. KATTHAGEN
   Neue Wege des Knochenersatzes.
   Orthop Prax, 1984, 20: 389 - 397
22. MOLINA, F., MONASTERIO, F.O.
   Mandibular elongation and remodelling by distraction: a farewell to major osteotomies
   Plast Reconstr Surg 1995, 96: 825 - 840.
23. PROCKOP, D.J.
   Marrow stromal cells as stem cells for nonhematopoietic tissues
   Science, 1997, 276: 71 - 74
24. PUELACHER, W.C., VACANTI, J.P., FERRARO, N.F., SCHLOO, B., VACANTI, C.A.
   Femoral shaft recon struction using tisuue-engineered growth of bone
   Int J Oral Maxillofac Surg, 1996, 25: 223 - 228.
25. RACHMIEL, A., LEVY, M., LAUFER, D.
   Lengthening of the mandible by distraction osteogenesis. Report of cases
   J Oral Maxillofac Surg 1995, 53: 838 - 846.
26. RAHN, B.A., PERREN, S.M
   Calcein blue as a fluorescent label in bone Experientia 26 1970, 26: 519-520
27. RAHN, B.A., PERREN, S.M
   Xylenol orange, a fluorochrome useful in polychrome sequential labeling of calcifying tissues Stain Technol 1971, 46: 125-129
28. REDDI, A.H., CUNNINGHAM, N.S.
   Initiation and promotion of bone differentiation by bone morphogenetic proteins.
   J Bone Min Res 1993, 8: 499.
29. SAMPATH, T.K., MALIAKAL, J.C., HAUSCHKA, P.V., JONES, W.K., SASAK, J.H., TUCKER, R.F., WHITE, K.H., COUGHLIN, J.E., TUCKER. M.M., PANG, R.H.L., CORBETT, C., ÖZYKAYNAK, E., OPPERMANN, H., RUEGER, D.C..
   Recombinant human osteogenic protein-1 (hOP-1) induces new bone formation in vivo with a specific activity comparable with natural bovine osteogenic protein and stimulates osteoblast proliferation and differentiation in vitro.
   J Biol Chem, 1992, 28: 20352.
30. SCHENK, R.K., BUSER, D., HARDWICK, W.R., DAHLIN, C.
   Healing pattern of bone regeneration in membrane-protected defects: A histologic study in the canine mandible.
   Int J Oral Maxillofac Impl 1994, 9: 13.
31. SCHLIEPHAKE, H., D. KLOSA, F.W. NEUKAM
   Automated microdensitometric quantification of bone ingrowth into porous implants.
   Int J Oral Maxillofac Impl, 1991, 6: 168 - 176
32. SCHLIEPHAKE, H., F. W. NEUKAM, A. LÖHR, D. HUTMACHER
   The use of basic fibroblast growth factor (bFGF) for enhancement of bone ingrowth into pyrolized bovine bone.
   Int J Oral Maxillofac Surg 1995, 24: 181 - 186
33. SCHLIEPHAKE, H., J. W. KNEBEL, M. TAUSCHER, F. MÜLLER, M. AUFDERHEIDE
   Extraction, cultivation and characterization of human osteoblast-like cells for tissue engineered growth of bone.
   Dtsch Z Mund Kiefer Gesichtschir 1996, 20: 90 - 94.
34. SCHLIEPHAKE, H., LANGNER, M.
   Reconstruction of the mandible by prefabricated autogenous bone grafts. An experimental study in minipigs
   Int J Oral Maxillofac Surg 1997a, 26: 244 - 252.
35. SCHLIEPHAKE, H., GROSS, G. KNEBEL, J.W., LAUBER, J., AUFDERHEIDE, M.
   Expression of mRNA for growth factors and bone specific markers in adult human osteoblast-like cell cultures
   Vortrag auf dem Joint Meeting of the German Society for Oral and Maxillofacial Surgery and the American Association of Oral and Maxillofacial Surgery, Hamburg, Mai 1997.
36. SCHLIEPHAKE, H., M. U. JAMIL, J. W. KNEBEL
   Reconstruction of the mandible using bioresorbable membranes and basic fibroblast growth factor (bFGF) in alloplastic scaffolds.
   J Oral Maxillofac Surg, 1997c, im Druck
37. SCHWEIBERER, L.
   Der heutige Stand der Knochentransplantation Der Chirurg, 1971, 42: 252 - 257
38. SIMION, M., PIATTELLI, A., SALVATO, A.
   Vertical ridge augmentation using a membrane technique associated with osseointegrated implants.
   Vortrag auf dem Bränemark Symposium, Gothenburg, 1995.
39. TAKAOKA, K., YOSHIKAWA, H., HASHIMOTO, J., ONO, K., MATSUI, M., NAKAZATO, H.
   Transfilter bone induction by chinese hamster ovary (CHO) cells transfected by DNA encoding bone morphogenetic protein-4, Clin Orthop Res, 1994, 300: 269 - 273.
40. TORIUMI, D.M., KOTLER, H.S., LUXENBERG, D.P., HOLTROP, M.E.,
   WANG, E.A.
   Mandibular reconstruction with a recombinant bone-inducing factor. Arch Otolaryngol 117: 1101, 1991.
41. URIST, M.R., HUO, Y.K., BROWNELL, A.G., HOHL, W.M., BUYSKE, J., LIETZKE, A., TEMPST, P., HUNKAPILLER, M., DELANGE, R.J.
   Purification of bovine bone morphogenetic protein by hydroxyapatite chromatography.
   Proc Natl Acad Sci USA 81: 371, 1984.
42. VACANTI, C.A., KIM, W., UPTON, J., VACANTI, M.P., MOONEY, D.,
   SCHLOO, B., VACANTI, J.P.
   Tissue-engineered growth of bone and cartilage, Transplant Proc, 1993, 25: 1019 - 1021
43. VILAMITJANA-AMEDEE, J., BAREILLE, R., ROUAIS, F., CAPLAN, A.I., ARMAND, M.-F.
   Human bone marrow stromal cells express an osteoblastic phenotype in culture
   In Vitro Cell Dev Biol, 1993, 29: 699 - 707
44. WANG, E. A., V. ROSEN, J. S. D'ALESSANDRO, M. BAUDUY, P. CORDES
   Recombinant human bone morphogenetic protein induces bone formation.
   Proc Natl Acad Sci USA, 1990, 87: 2220

Boris-Lawrie K. A. And Temin H. M.
Recent advances in retrovirus technology Current opinion in Genetics and Development 1993, 3. 102 - 109

Yeh, P. and Perricaudet, M.
Advances in adenoviral vectors: from genetic engineering to their biology.
Faseb J. 1997, 11, 615-623

Ali, M., Lemoine, N. R. And Ch. J. A. Ring.
The use of DNA viruses as vectors for gene therapy. Gene Therapy 1994, 1, 367-384.

Kübeler, N., Steveling, H., Reuter, J., Bialas, M., Urist, M. R. Auffüllung von Kieferzysten mit autolysiertem, Antigen extrahiertem, allogenem Knochen (AAA-Bone), Dtsch. Z. Mund Kiefer GesichtsChir 1993, 17, 95 - 97.

Röttgen P. And Collins J.
A human pancreatic secretory trypsin inhibitor presenting a hypervariable highly constrained ipitope via monovalent phagemid display
Gene 1995, 164, 243-250.

De Kruif J. Trestappen, L., Boel, E., and Logtenberg, T. Rapid selection of cell subpopulation-specific human monoclonal antibodies from a synthetic phage antibody library Proc. Natl. Acad. Sci. USA, 1995, 92, 3938- 3942.

Nissim, A., Hoogenboom, H., R., Tomlinson, I, M., Flynn, G., Midgley, C., Lane, D., and Winter, G.
Antibody fragments from a single pot phage display library as immunochemical reagents.
EMBO J. 1994, 13, 692- 698.

Kadiyla, S., Jaiswal, N. and Bruder, S.
Cuture expanded, bone Marrow-derived mesenchymal stem cells can regenerate a critical-sized segmental bone defect.
Tissue Engineering. 1997, 3 , 173 - 185.

Prockop, D.,J.
Marrow stromal cells as stem cells for nonhematopoietic tissues. Science,1997, 276, 71 - 74
18

## Patentansprüche

1. Knochenmatrix, dadurch gewinnbar, daß man
(a) von einem Explantat somatischer Zellen der Haut, des Knorpels, des Knochens oder des Zahnapparates ausgeht und osteogene Vorläuferzellen gewinnt oder von einer osteogenen Zellinie ausgeht,
(b) gegebenenfalls die gewonnenen osteogenen Vorläuferzellen oder die osteogene Zellinie in vitro expandiert,
(c) gemäß (a) gewonnene oder gemäß (b) expandierte osteogene Vorläuferzellen oder die gemäß (a) eingesetzte oder gemäß (b) expandierte Zellinie sich vermehren läßt und gegebenenfalls osteogene Zellen gewinnt,
(d) getrennt von Maßnahme (c) gemäß (a) gewonnene oder gemäß (b) expandierte osteogene Zellen oder die gemäß (a) eingesetzte oder gemäß (b) expandierte Zellinie in vitro extrazelluläre Matrix synthetisieren läßt und Knochenmatrix gewinnt,
(e) die synthetisierte Matrix gegebenenfalls von Zellen befreit und zellfreie Knochenmatrix gewinnt, und
(f) die gewonnene, gegebenenfalls zellfreie Knochenmatrix gegebenenfalls erneut mit osteogenen Zellen besiedelt, die gemäß (c) gewonnen worden sind, und durch osteogene Zellen besiedelte Knochenmatrix gewinnt.

2. Knochenmatrix nach Anspruch 1, dadurch gewinnbar, daß man bei Stufe (c) mit Hilfe
(i) einer Zugabe von Wachstumsfaktoren und/oder von Wachstumsfaktor-Rezeptoren und/oder
(ii) einer genetischen Transfektion mit Genen einer oder mehrerer der Komponenten gemäß (i) vermehrt.

3. Knochenmatrix nach Anspruch 2, dadurch gewinnbar, daß man als Wachstumsfaktoren gemäß (i) Fibroblast Growth Factors (FGFs), Epidermal Growth Factors (EGFs), insbesondere VEGFs, oder Parathormone (PTHs) und/oder als Wachstums-Rezeptoren FGFRs, VEGFRs oder PTHRs und/oder gemäß (ii) deren Gene verwendet.

4. Knochenmatrix nach einem der vorhergehenden Ansprüche, dadurch gewinnbar, daß man bei Stufe (d) mit Hilfe
(i) einer Zugabe von Wachstumsfaktoren, insbesondere Bone Morphogenetic Proteins (BMPs), und/oder Wachstumsfaktoren-Rezeptoren, insbesondere BMPRs, und/oder BMPR-Signalmolekülen (Smads) und/oder
(ii) einer genetischen Transfektion mit Genen einer oder mehrerer der Komponenten gemäß (i) extrazelluläre Matrix synthetisiert.

5. Knochenmatrix nach einem der vorhergehenden Ansprüche, dadurch gewinnbar, daß man bei Stufe (e) die die Knochenmatrix besiedelnden Zellen beläßt oder in ihrer Vermehrung behindert, insbesondere durch Bestrahlung oder durch chemische Behandlung.

6. Knochenmatrix nach einem der Ansprüche 1 bis 4, dadurch gewinnbar, daß man bei Stufe (e) die die Knochenmatrix besiedelnden Zellen entfernt, indem man die Zellen enzymatisch ablöst oder einfriert und durch Waschen entfernt.

7. Knochenmatrix nach einem der vorhergehenden Ansprüche, dadurch gewinnbar, daß man bei Stufe (f)
(i) zellfreie Knochenmatrix, die mit Hilfe von autogenen osteogenen Vorläuferzellen des Menschen gewonnen worden ist, mit osteogenen Zellen xenogener oder allogener Herkunft besiedelt oder
(ii) zellfreie Knochenmatrix, die mit Hilfe von xenogenen oder allogenen osteogenen Vorläuferzellen gewonnen worden ist, mit osteogenen Human-Zellen autogener Herkunft besiedelt oder
(iii) zellfreie Knochenmatrix, die mit Hilfe von autogenen osteogenen Vorläuferzellen des Menschen gewonnen worden ist, mit osteogenen Human-Zellen autogener Herkunft besiedelt.

8. Knochenmatrix nach einem der vorhergehenden Ansprüche, dadurch gewinnbar, daß man bei Stufe (a) von Explantaten des Beckenkamms ausgeht.

9. Knochenmatrix nach einem der vorhergehenden Ansprüche, dadurch gewinnbar, daß man bei Stufe (c) oder (f) die Zellen mit einem Marker versieht, insbesondere mit einem TAG-Gen transfiziert, das insbesondere ein Green-Light-Fluorescence-Protein oder β-Galaktosidase kodiert.

10. Therapeutische Zubereitung, **gekennzeichnet durch** eine Knochenmatrix gemäß einem der vorhergehenden Ansprüche.

11. Zubereitung nach Anspruch 10 in Form einer Salbe, einer Emulsion oder eines Pflasters.

12. Kit, **gekennzeichnet durch** eine Knochenmatrix mit osteogenen Zellen gemäß einem der Ansprüche 1 bis 9 zum Testen von exogenen Faktoren, die die Bildung und Differenzierung von Matrix und Zellen beeinflussen.

13. Verwendung von Knochenmatrix gemäß einem der Ansprüche 1 bis 9 zur Herstellung eines autogenen, xenogenen oder allogenen Implantatmaterials.

## Claims

1. Bone matrix, obtainable by
(a) starting from an explant of somatic cells of skin, of cartilage, of bone or of teeth and obtaining osteogenic precursor cells, or starting from an osteogenic cell line,
(b) optionally expanding the resulting osteogenic precursor cells or the osteogenic cell line *in vitro*,
(c) allowing osteogenic precursor cells obtained according to (a) or expanded according to (b), or the cell line used according to (a) or expanded according to (b), to multiply, and optionally obtaining osteogenic cells,
(d) separately from measure (c), allowing osteogenic cells obtained according to (a) or expanded according to (b), or the cell line used according to (a) or expanded according to (b), to synthesise extracellular matrix *in vitro*, and obtaining bone matrix,
(e) optionally freeing the synthesised matrix of cells, and obtaining cell-free bone matrix, and
(f) optionally re-populating the resulting optionally cell-free bone matrix with osteogenic cells obtained according to (c), and obtaining bone matrix populated with osteogenic cells.

2. Bone matrix according to claim 1, obtainable by carrying out the multiplication in step (c) with the aid of
(i) the addition of growth factors and/or of growth-factor receptors and/or
(ii) genetic transfection using genes of one or more components according to (i).

3. Bone matrix according to claim 2, obtainable by using as growth factors according to (i) fibroblast growth factors (FGFs), epidermal growth factors (EGFs), especially VEGFs, or parathormones (PTHs) and/or by using as growth receptors FGFRs, VEGFRs or PTHRs and/or by using genes thereof according to (ii).

4. Bone matrix according to any one of the preceding claims, obtainable by synthesising extracellular matrix in step (d) with the aid of
(i) the addition of growth factors, especially bone morphogenetic proteins (BMPs), and/or of growth-factor receptors, especially BMPRs and/or BMPR signal molecules (Smads), and/or
(ii) genetic transfection using genes of one or more components according to (i).

5. Bone matrix according to any one of the preceding claims, obtainable by leaving the cells that populate the bone matrix in step (e) or by hindering the multiplication thereof, especially by irradiation or by chemical treatment.

6. Bone matrix according to any one of claims 1 to 4, obtainable by removing in step (e) the cells that populate the bone matrix, by enzymatically removing or freezing the cells and removing them by washing.

7. Bone matrix according to any one of the preceding claims, obtainable by, in step (f),
(i) populating cell-free bone matrix obtained with the aid of autogenic osteogenic precursor cells of humans with osteogenic cells of xenogenic or allogenic origin or
(ii) populating cell-free bone matrix obtained with the aid of xenogenic or allogenic osteogenic precursor cells with osteogenic human cells of autogenic origin or
(iii) populating cell-free bone matrix obtained with the aid of autogenic osteogenic precursor cells of humans with osteogenic human cells of autogenic origin.

8. Bone matrix according to any one of the preceding claims, obtainable by starting from explants of the iliac crest in step (a).

9. Bone matrix according to any one of the preceding claims, obtainable by providing the cells with a label in step (c) or (f), especially by transfecting them with a TAG gene which codes especially for a green-light-fluorescence protein or β-galactosidase.

10. Therapeutic preparation, **characterised by** a bone matrix according to any one of the preceding claims.

11. Preparation according to claim 10 in the form of an ointment, an emulsion or a plaster.

12. Kit, **characterised by** a bone matrix having osteogenic cells according to any one of claims 1 to 9, for testing exogenic factors which affect the formation and differentiation of matrix and cells.

13. Use of bone matrix according to any one of claims 1 to 9 in the production of an autogenic, xenogenic or allogenic implant material.

## Revendications

1. Matrice osseuse, que l'on peut obtenir de la façon suivante :
a) on part d'un explant de cellules somatiques de peau, de cartilage, d'os ou d'appareil dentaire et l'on en prélève des cellules précurseurs ostéogènes, ou bien l'on part d'une lignée cellulaire ostéogène ;
b) le cas échéant, on provoque l'expansion cellulaire *in vitro* des cellules précurseurs ostéogènes obtenues ou de la lignée cellulaire ostéogène ;
c) on fait croître soit les cellules précurseurs ostéogènes obtenues lors de l'étape (a) ou expansées lors de l'étape (b), soit la lignée cellulaire de laquelle on part lors de l'étape (a) ou qui a été expansée lors de l'étape (b), et l'on prélève éventuellement des cellules ostéogènes ;
d) dans cette étape bien distincte de l'étape (c), on fait synthétiser *in vitro*, soit par les cellules ostéogènes obtenues lors de l'étape (a) ou expansées lors de l'étape (b), soit par la lignée cellulaire de laquelle on part lors de l'étape (a) ou qui a été expansée lors de l'étape (b), une matrice extracellulaire, pour obtenir une matrice osseuse ;
e) on débarrasse éventuellement la matrice synthétisée des cellules qu'elle contient, pour obtenir une matrice osseuse sans cellules ;
f) et le cas échéant, l'on peuple à nouveau la matrice osseuse éventuellement sans cellules obtenue, avec des cellules ostéogènes obtenues lors de l'étape (c), pour obtenir une matrice osseuse peuplée de cellules ostéo-gènes.

2. Matrice osseuse conforme à la revendication 1, qu'on peut obtenir en provoquant la croissance cellulaire, lors de l'étape (c), à l'aide :
i) d'une addition de facteurs de croissance et/ou de récepteurs de facteurs de croissance,
ii) et/ou d'une transfection génétique effectuée avec des gènes d'un ou de plusieurs des composants mentionnés en (i).

3. Matrice osseuse conforme à la revendication 2, qu'on peut obtenir en utilisant selon (i), comme facteurs de croissance, le FGF (facteur de croissance des fibroblastes), l'EGF (facteur de croissance épidermique), en particulier le VEGF, ou la PTH (parathormone), et/ou, comme récepteurs de facteurs de croissance, des récepteurs FGF-R, VEGF-R ou PTH-R, et/ou en utilisant selon (ii) les gènes de ces substances.

4. Matrice osseuse conforme à l'une des revendications précédentes, qu'on peut obtenir en provoquant la synthèse d'une matrice extracellulaire, lors de l'étape (d), à l'aide
i) d'une addition de facteurs de croissance, en particulier de BMP (protéine morphogénétique osseuse), et/ou de récepteurs de facteurs de croissance, en particulier de BMP-R, et/ou de molécules signaux pour BMP-R (Smad),
ii) et/ou d'une transfection génétique effectuée avec des gènes d'un ou de plusieurs des composants mentionnés en (i).

5. Matrice osseuse conforme à l'une des revendications précédentes, qu'on peut obtenir en laissant présentes et intactes, lors de l'étape (e), les cellules qui peuplent la matrice osseuse, ou en empêchant leur croissance, en particulier par irradiation ou par traitement chimique.

6. Matrice osseuse conforme à l'une des revendications 1 à 4, qu'on peut obtenir en éliminant, lors de l'étape (e), les cellules qui peuplent la matrice osseuse, c'est-à-dire en libérant ces cellules par voie enzymatique ou en les congelant et en les extrayant par lavage.

7. Matrice osseuse conforme à l'une des revendications précédentes, qu'on peut obtenir en peuplant, lors de l'étape (f),
i) une matrice osseuse sans cellules obtenue à l'aide de cellules précurseurs ostéogènes autogènes humaines, avec des cellules ostéogènes d'origine allogène ou xénogène,
ii) une matrice osseuse sans cellules obtenue à l'aide de cellules précurseurs ostéogènes allogènes ou xénogènes, avec des cellules ostéogènes humaines d'origine autogène,
iii) ou une matrice osseuse sans cellules obtenue à l'aide de cellules précurseurs ostéogènes autogènes humaines, avec des cellules ostéogènes humaines d'origine autogène.

8. Matrice osseuse conforme à l'une des revendications précédentes, qu'on peut obtenir en partant, lors de l'étape (a), d'explants de crête iliaque.

9. Matrice osseuse conforme à l'une des revendications précédentes, qu'on peut obtenir en munissant les cellules d'un marqueur lors de l'étape (c) ou (f), en particulier par transfection avec un gène TAG qui code, en particulier, une protéine à fluorescence en lumière verte ou la β-galactosidase.

10. Préparation thérapeutique, **caractérisée par** une matrice osseuse conforme à l'une des revendications précédentes.

11. Préparation conforme à la revendication 10, qui se présente sous la forme d'une pommade, d'une émulsion ou d'un pansement.

12. Trousse **caractérisée par** une matrice osseuse associée à des cellules ostéogènes, conforme à l'une des revendications 1 à 9, destinée à tester des facteurs exogènes qui influencent la formation et la différentiation de la matrice et des cellules.

13. Emploi d'une matrice osseuse conforme à l'une des revendications 1 à 9 pour la fabrication d'un matériau d'implant autogène, xénogène ou allogène.
